# EUROPÄISCHE PATENTSCHRIFT

(11) **EP 1 788 114 B1**
(45) Veröffentlichungstag und Bekanntmachung des Hinweises auf die Patenterteilung: **16.05.2012**
(21) Anmeldenummer: 06023391.3
(22) Anmeldetag: 10.11.2006
(51) Int. Cl.: A61L 27/30, A61L 33/02, C23C 14/06, A61B 19/00

(54) **Verfahren zur Herstellung einer amorphen Kohlenstoffschicht**
Method for forming amorphous carbon film
Procédé de fabrication d'une couche de carbone amorphe

(30) Priorität: 17.11.2005 DE 102005054703; 17.11.2005 DE 102005054704
(43) Veröffentlichungstag der Anmeldung: 23.05.2007
(73) Patentinhaber: Karlsruher Institut für Technologie, 76131 Karlsruhe (DE)
(72) Erfinder: Stüber, Michael, Dr., 76829 Landau (DE); Niederberger, Lorenz, 76135 Karlsruhe (DE); Leiste, Harald, Dr., 76356 Weingarten (DE); Ulrich, Sven, Dr., 76297 Stutensee (DE); Welle, Alexander, Dr., 68723 Schwetzingen (DE); Fischer, Harald, Dr., 76356 Weingarten (DE)

(56) Entgegenhaltungen:
- DE-C1- 19 938 945
- US-A- 4 524 106
- PENG X L ET AL: "Surface roughness of diamond-like carbon films prepared using various techniques" SURFACE & COATINGS TECHNOLOGY ELSEVIER SWITZERLAND, Bd. 138, Nr. 1, 2. April 2001 (2001-04-02), Seiten 23-32, XP002476201 ISSN: 0257-8972

## Beschreibung

Die Erfindung betrifft ein Verfahren zur Herstellung einer amorphen Kohlenstoffschicht mit einer thrombozytenabweisenden Eigenschaft, die einen Gehalt an Wasserstoff von höchstens 5 Atom% aufweist, auf einem Substrat.

Unmittelbar nach dem erfolgten Kontakt eines medizinischen Instruments oder Implantats mit Blut wird an dessen Oberfläche eine Schicht aus Proteinen aus dem Blutplasma adsorbiert, die nachfolgend insbesondere mit der Aktivierung des Gerinnung- und Komplementsystems des Bluts, mit inflammatorischen Reaktionen und mit Zellanhaftungen assoziiert wird.

Aus der Anlagerung der Adsorptionsproteine HMWK *(High Molecular Weight Kiniogen)* oder Albumin resultiert eine reduzierte Adhäsion und Aktivierung von Blutzellen (Thrombozyten, Monozyten, Granulozyten). Die Gewebeantwort auf ein Substrat, die ausschlaggebend für die Frage und Dauer dessen Einsatzes (Langzeit-Stabilität und Biokompatibilität) ist, hängt nicht nur von dessen Oberfläche, sondern auch von der Schicht adsorbierter Proteine ab. Für die Zusammensetzung dieser Proteinschicht sind das Substrat und die Beschaffenheit seiner Oberfläche verantwortlich.

Das Maß der Thrombozytenabweisung bzw. -anlagerung (Thrombogenität) auf der Oberfläche eines medizinischen Instruments oder eines Implantats stellt eines der wichtigsten Kriterien zur Beurteilung der Bio- bzw. der Hämokompatibilität dar. Die Freisetzung von thrombogenen Substanzen kann eine massive Gerinnungsstörung (Hyperkoagulabilität), d. h. eine vermehrte Gerinnbarkeit des Blutes bis hin zu thrombotischen Verschlüssen verursachen. Allein in Deutschland sterben jährlich rund 30000 Menschen an den Folgen einer Lungenembolie.

Aus P. Yang, N. Huang, Y.X. Leng, J.Y. Chen, R.K.Y. Fu, S.C.H. Kwok, Y. Leng und P.K. Chu, Activation of platelets adhered on amorphous hydrogenated carbon (a-C:H) films synthesized by plasma immersion ion implantation-deposition (PIII-D), Biomaterials 24 (2003), S. 2821-2829, ist ein Verfahren zur Herstellung von amorphen Kohlenstoffschichten mittels PIII-D *(Plasma immersion ion implantation and deposition)* bekannt.

S.C.H. Kwok, J. Wang und P.K. Chu stellen in Surface energy, wettability, and blood compatibility of phosphorus doped diamond-like carbon films, Diamond. Relat. Mater. 14 (2005), S. 78-85, ein Verfahren zur Herstellung von mit Phosphor dotierten amorphen Kohlenstoffschichten mittels PIII-D vor.

Aus T. Saito, T. Hasebe, S. Yohena, Y. Matsuoka, A. Kamijo, K. Takahashi und T. Suzuki, Antithrombogenicity of fluorinated diamond-like carbon films, Diamond. Relat. Mater. 14 (2005), S. 1116-1119, ist ein Verfahren zur Herstellung von mit Fluor dotierten amorphen Kohlenstoffschichten mittels chemischer Abscheidung aus der Dampfphase (*Chemical Vapor Deposition,* CVD) mit C₂H₂ und C₂F₆ bekannt.

T.I.T. Okpalugo, A.A. Ogwu, P.D. Maguire und J.A.D. McLaughlin zeigen in Platelet adhesion on silicon modified hydrogenated amorphous carbon films, Biomaterials 25 (2004), S. 239-245, ein Verfahren zur Herstellung von mit Silizium dotierten amorphen Kohlenstoffschichten mittels plasmaverstärkter chemischer Abscheidung aus der Dampfphase *(Plasma Enhanced Chemical Vapor Deposition,* PECVD).

M.I. Jones, I.R. McColl, D.M. Grant, K.G. Parker und T.L. Parker offenbaren in Protein adsorption and platelet attachment and acivation on TiN, TiC and DLC coatings on titanium for cardiovascular applicatons, J. Biomed. Mater. Res. 52 (2000), S. 413-21, ein Verfahren zur Herstellung von amorphen Kohlenstoffschichten mittels Ionenplattieren *(Ion-plating)* mit Acetylen.

US4524106 offenbart ein Verfahren zur Herstellung einer amorphen wasserstofffreien Kohlenstoffschicht, mit den Schritten:
a) Polieren und Reinigen des Metallsubstrats,
b) Positionieren des Substrats in einer Prozesskammer einer Magnetronsputteranlage und Evakuierung der Prozesskammer,
c) Plasmaätzen des Substrats,
d) Abscheiden der amorphen Kohlenstoffschicht von einem Graphit -Target auf das so behandelte Substrat bei Raumtemperatur, einem Argon -Partialdruck von 0,8 Pa und einer Sputterleistung von 1,24 W/cm².

Die Aufgabe der Erfindung ist es, ein Verfahren zur Herstellung einer amorphen Kohlenstoffschicht anzugeben, mit dem sich das Maß der Thrombogenität, d.h. der Thrombozytenabweisung , auf einer Oberfläche eines Substrats einstellen lässt.

Diese Aufgabe wird durch die Schritte des Anspruchs 1 gelöst. Die Unteransprüche geben jeweils vorteilhafte Ausgestaltungen der Erfindung.

Das erfindungsgemäße Verfahren zur Herstellung der amorphen Kohlenstoffschicht erfolgt gemäß den folgenden Schritten a) bis d) :
a) Vorbehandeln und Reinigen des Substrats,
b) Positionieren des Substrats in einer Prozesskammer,
c) Plasmaätzen des Substrats und
d) Beschichten des Substrats.

Das bereitgestellte Substrat wird zunächst gemäß Schritt a) einer Vorbehandlung unterzogen.

Als Substrat eignen sich alle Materialien, die intra- oder extrakorporal im direkten Kontakt mit Gewebe stehen können. Hierzu zählen Metalle oder Legierungen, bevorzugt Ti, Ti6A14V, NiTi, TiAl, Gold, legierte oder unlegierte (Edel-)Stähle. Weiterhin eignen sich glasartige oder keramische Substrate wie z.B. Glaskohlenstoff (Sigradur-G, Sigradur-K), SiC, Si₃N₄, Al₂O₃ oder ZrO₂. Aufgrund der geringen Verfahrenstemperatur von 50 °C bis 200°C sind auch Substrate aus Kunststoffen (Polymeren), insbesondere Teflon (PTFE), Polyetheretherketon (PEEK), Polymethylmethacrylat (PMMA), Acrylnitrilmethylmethacrylat (AMMA), Polyamid (PA), Polyimid (PI), Polycarbonat (PC), Polyvinylchlorid (PVC), Polypropylen (PP), Polyurethan (PU) oder Polyethylen (PE) geeignet. Das Substrat selbst kann fest oder lösbar mit einer weiteren Unterlage verbunden sein.

Das Substrat wird geschliffen und poliert, um eine möglichst homogene Oberfläche zu erzeugen, und gereinigt. Insbesondere sollen im Lichtmikroskop weder Materialanhäufungen, die durch unterschiedlich harte Komponenten z. B. in Legierungen gebildet werden, noch eine Bildung von Poren, eine Oberflächenschädigung oder Riefen durch heraus gelöste Komponenten erkennbar sein.

Nach der Vorbehandlung wird das Substrat gemäß Schritt b) in eine Prozesskammer einer Magnetronsputteranlage eingebracht und in der Prozesskammer für die nachfolgenden Verfahrensschritte ausgerichtet (positioniert). Anschließend erfolgt eine Evakuierung der Prozesskammer. Die Evakuierung wird vorzugsweise so lange durchgeführt, bis die Prozesskammer (Rezipient) nur noch einen Restgasdruck von 1·10⁻⁴ Pa bis 4.10⁻⁴ Pa aufweist.

In der Prozesskammer wird das Substrat zunächst gemäß Schritt c) einem Plasmaätzverfahren unterworfen. Als Arbeitsgas dient vorzugsweise Argon bei einem Argon-Partialdruck von 0,2 Pa bis 2,0 Pa.

Schließlich erfolgt gemäß Schritt d) das Abscheiden der amorphen Kohlenstoffschicht von einem Graphit-Traget auf das vorbehandelte, positionierte und plasmageätzte Substrat. Hierzu wird ein Argon-Partialdruck von 0,2 Pa bis 2,0 Pa eingestellt.

Zur Herstellung einer thrombozytenanreichernden Kohlenstoffschicht erfolgt das Abscheiden vorzugsweise bei einer Sputterleistung pro Flächeneinheit von 0,57 bis 7,91 W/cm², während zur Herstellung einer thrombozytenabweisenden Kohlenstoffschicht das Abscheiden vorzugsweise bei einer Sputterleistung pro Flächeneinheit von 7,91 bis 15,58 W/cm² erfolgt.

Die mit dem Verfahren hergestellte thrombozytenanreichernde Schicht besteht aus einer amorphen Kohlenstoffschicht, deren Oberflächenenergie unterhalb eines Schwellenwerts von 34,4 mN/m liegt, wobei die Oberflächenenergie einen Wert bis einschließlich 27,2 mN/m annehmen kann. Der polare Anteil der Oberflächenenergie beträgt hierbei von 9 mN/m bis 15 mN/m. Sämtliche Werte für die Oberflächenenergie, die in diesem Dokument angegeben sind, wurden nach dem Verfahren von D.K. Owens, R.C. Wendt, J. Appl. Polym. Sci. (1969), S. 1741-1747, bestimmt.

Die mit dem erfindungsgemäßen Verfahren hergestellte thrombozytenabweisende Schicht besteht aus einer amorphen Kohlenstoffschicht, deren Oberflächenenergie oberhalb eines Schwellenwerts von 34,4 mN/m liegt, wobei die Oberflächenenergie einen Wert bis einschließlich 47,7 mN/m annehmen kann. Der polare Anteil der Oberflächenenergie beträgt hierbei von 5 mN/m bis 8,9 mN/m.

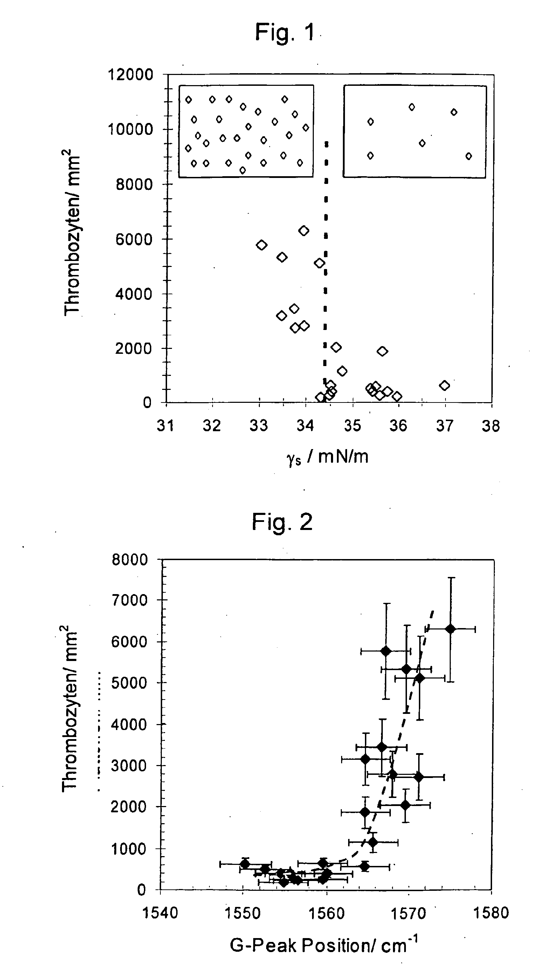

Die vorliegende Erfindung macht Gebrauch von dem von den Erfindern aufgefundenen und in der **Fig. 1** dargestellten Zusammenhang zwischen der Oberflächenenergie und dem Grad der Adhärenz von Thrombozyten auf erfindungsgemäßen Schichten. Die Oberflächenenergie besitzt bei ca. 34,4 mN/m eindeutig einen kritischen Wert für das Maß der Adhärenz von Thrombozyten auf der Oberfläche. Erst die Aufdeckung dieses Sachverhalts ermöglicht es, auf einfache und wirtschaftliche Art und Weise eine erfindungsgemäß beschichtete Oberfläche eines Substrats gezielt bezüglich ihres Verhaltens zur Thrombogenität einzustellen.

Im Zusammenhang mit der Aufdeckung dieses Sachverhalts steht die Position der G-Bande eines Raman-Spektrums im sichtbaren Bereich (Anregungswellenlänge von 514,5 nm), die in **Fig. 2** als Funktion der Zahl anhaftender Thrombozyten aufgetragen ist. Für eine thrombozytenanreichernde Schicht befindet sich die G-Bande im Bereich von 1565 cm⁻¹ bis 1580 cm⁻¹, während sich für eine thrombozytenabweisende Schicht die G-Bande im Bereich von 1550 cm⁻¹ bis 1560 cm⁻¹ befindet.

Das Beschichtungsverfahren wird vorzugsweise bei einer Temperatur von 50 °C bis 200 °C, besonders bevorzugt von 70 °C bis 150 °C, durchgeführt. Das auf diese Weise mit einer amorphen Kohlenstoffschicht versehene Substrat kann nach erfolgtem Druckausgleich und Abkühlen auf höchstens 60 °C, vorzugsweise auf Raumtemperatur, der Beschichtungsanlage entnommen werden.

Die mit dem erfindungsgemäßen Verfahren hergestellte Schicht besitzt einen Gehalt an Wasserstoff von höchstens 5 Atom%, der mittels Kernreaktionsanalyse *(Nuclear Reaction Analysis,* NRA) bestimmt werden kann. Damit gilt diese Schicht quasi als wasserstofffrei.

Die erfindungsgemäß hergestellte Schicht weist eine Schichtdicke von 50 nm bis 10 µm, bevorzugt von 100 nm bis 5 µm, besonders bevorzugt von 500 nm bis 2 µm auf.

Die Härte der erfindungsgemäß hergestellten Schicht beträgt von 5 GPa bis 40 GPa. Der mit Elektronenenergieverlustspektroskopie *(Electron Energy Loss Spectroscopy,* EELS) bestimmte Sp³⁻Bindungsanteil der erfindungsgemäßen Schicht nimmt einen Wert im Bereich von 0% bis 30% an.

Die Rauheit der erfindungsgemäß hergestellten Schicht beträgt bei der Betrachtung des arithmetischen Mittenrauhwerts Rₐ von 3 nm bis 23 nm, der Glättungstiefe Rₚ von 16 nm bis 200 nm, des quadratischen Mittenrauhwerts R_{q} von 6 nm bis 27 nm, der Differenz Rₜ zwischen der höchsten Spitze zur tiefsten Tiefe von 29 nm bis 170 nm und der gemittelten Rauhtiefe R_{z(ISO)} von 20 nm bis 190 nm.

Der (advancing) Benetzungswinkel einer thrombozytenanreichernden Schicht für H₂O_{dest} nimmt einen Wert von 78° bis 90° sowie für Formamid von 60° bis 65° an, während der (advancing) Benetzungswinkel einer thrombozytenabweisenden Schicht für H₂O_{dest} einen Wert von 65° bis 77° sowie für Formamid von 50° bis 59° annimmt.

Das erfindungsgemäße Verfahren erlaubt es, verschiedene Geometrien, sowohl in zwei Dimensionen (planar) als auch in drei Dimensionen (räumlich) zu beschichten.

In einer besonderen Ausgestaltung weist eine thrombozytenanreichernde Schicht eine Adhärenz von Thrombozyten von mindestens 3000 Plättchen (Thrombozyten) pro mm², bevorzugt von mindestens 5000 Plättchen (Thrombozyten) pro mm² auf.

In einer besonderen Ausgestaltung weist eine thrombozytenabweisende Schicht eine Adhärenz von Thrombozyten von höchstens 2000 Plättchen (Thrombozyten) pro mm², bevorzugt von höchstens 1000 Plättchen (Thrombozyten) pro mm² auf.

Das vorliegende Verfahren eignet sich zur Herstellung einer thrombozytenanreichernden Schicht als Trennmedium für Thrombozyten aus einer Suspension, die Thrombozyten enthält, insbesondere Blut. Hierzu werden die Thrombozyten selektiv an einer oder mehreren erfindungsgemäßen Schichten angelagert (aktiviert), um diese von sämtlichen Stoffen zu trennen, die biologische Aktivität aufweisen. Auf diese Weise lassen sich Thrombozyten oder Thrombozytenüberstände gewinnen.

Der Thrombozytenüberstand von aktivierten Thrombozyten kann einerseits zur Gewinnung von Ausgangsmaterialien zur Aufreinigung von biologischen Molekülen verwendet werden, wie z. B. PDGF (*Platelet-derived growth factor*), dem Von Willebrand-Faktor, TGFβ (*transforming growth factor β*), Plasminogen, Faktor XIII und weitere. Andererseits kann der Überstand aktivierter Thrombozyten oder eine Fraktion dieser Überstandes direkt zur Herstellung eines Pharmazeutikums oder eines Blutproduktes z. B. zur Wundheilung oder in der Kosmetik verwendet werden.

Die gebundenen und gereinigten Thrombozyten lassen sich mit Hilfe bekannter Aktivatoren suspendieren. Die gereinigten aktivierten suspendierten Thrombozyten werden z. B. mit Ultraschall oder anderen physischen Methoden lysiert und die bekannten Inhaltstoffe, insbesondere die biologisch aktiven Proteine und Peptide aus den Granula zur Verwendung als Pharmazeutikum, Blutprodukt oder Kosmetikum mittels Zentrifugation oder Filtration aufgereinigt.

Die gebundenen und suspendierten Thrombozyten eignen sich zur Wirkstoffssuche *(Screening)* in der pharmazeuischen oder kosmetischen Industrie, z. B. zur Suche nach neuen Thrombozytenaggregationshemmern oder dienen zur toxikologischen oder pharmakologischen Untersuchung von bekannten Wirkstoffen. Die Wirkstoffe können niedermolekular sein bzw. Proteine oder Peptide umfassen. Weiterhin dienen die Thrombozyten zur Untersuchung der Vorgänge bei der primären und sekundären Hämostase, insbesondere auf molekularer Ebene.

Das vorliegende Verfahren eignet sich weiterhin zur Herstellung einer thrombozytenanreichernden Schicht für ein analytisches Test-Kit, das sich dafür eignet, z. B. für ein Individuum für diagnostische oder prognostische Zwecke den Inhalt von Thrombozytengranulaten zu bestimmen.

Die vorliegende Erfindung betrifft die Herstellung einer thrombozytenabweisenden Schicht für medizinische Instrumente oder Implantate, die als Substrat dienen und die jeweils zumindest teilweise mit mindestens einer oder mehreren thrombozytenabweisenden Schichten beschichtet sind. Dazu gehören Kurz- oder Langzeit-Implantate, insbesondere Stents, Knochenanker, Venenfilter, Gefäßclips, Aneurysmenstents, koronare oder venöse Klappen und Klappensysteme, Zahnregulierdrähte oder mechanische Bypassverbindungen. Ebenso eignen sich medizinische Instrumente für die offene oder die minimalinvasive Chirurgie wie z.B. Scheren, Zangen, Retraktoren, Skalpelle, Nadeln, Biopsie-Instrumente, Katheter oder Nadelhalter als Substrat.

Die Erfindung wird im Folgenden an Hand von Ausführungsbeispielen mit Hilfe der Abbildungen näher erläutert. Es zeigen:
- **Fig.1**: Thrombozyten pro mm² als Funktion der Oberflächen-energie von thrombozytenabweisenden und thrombozyten-anreichernden Schichten.
- **Fig. 2**: Thrombozyten pro mm² als Funktion der der Position der G-Ramanbande von thrombozytenabweisenden und thrombozytenanreichernden Schichten.

**Fig. 1** zeigt eine Darstellung des Zusammenhanges zwischen der Oberflächenenergie und der Thrombozytenadhärenz von thrombozytenanreichernden sowie von thrombozytenabweisenden Schichten. Bei einer Oberflächenenergie unterhalb des aufgefundenen Schwellenwertes von 34,4 mN/m wird eine starke Thrombozytenadhärenz (Thrombozytenanreicherung), bei einer Oberflächenenergie oberhalb des aufgefundenen Schwellenwertes von 34,4 mN/m dagegen eine schwache Thrombozytenadhärenz (Thrombozytenabweisung) beobachtet.

In **Fig. 2** ist die Abhängigkeit der Zahl anhaftender Thrombozyten von der Position der G-Ramanbande dargestellt. Raman-Spektroskopie ist sehr empfindlich auf kleine Anteile an Sp²-Bindung in Diamant bzw. amorphen Kohlenstoffschichten. Reiner Diamant, der ausschließlich Sp³-konfigurierte Kohlenstoff-Bindungen besitzt, weist eine diskrete Linie bei 1332 cm⁻¹, reiner Graphit dagegen, bei dem ausschließlich Sp²-konfigurierte Kohlenstoff-Bindungen auftreten, bei 1580 cm⁻¹ auf, die im Falle des Graphits als G-Bande *(Graphite-Peak)* bezeichnet wird. Die Verschiebung der Position der G-Bande zu höheren Wellenzahlen ist mit der vermehrten Anlagerung von Thrombozyten, die Verschiebung der Position der G-Bande zu niedrigeren Wellenzahlen mit der verringerten Anlagerung von Thrombozyten korreliert.

Die Raman-Spektren wurden mit einem kommerziellen Raman-Spektrometer bei einer Anregungswellenlänge von 514,5 nm (ArgonIonen-Laser) aufgezeichnet. Vor der Messung erfolgte eine Kalibrierung an einem Silizium-Wafer mit gut definierter Oberfläche, der eine einzige diskrete Bande bei 520 cm besitzt. Die Proben wurden vor der Messung mit Isopropanol gesäubert. Die aufgenommenen Spektren wurden unter Verwendung einer linearen Grundlinie mit zwei Gauss-Peaks angepasst, dabei wurden die Parameter Bandenhöhe, -breite und -position während der iterativen Anpassung nicht fixiert. Die Kurvenanpassung konvergierte nach ca. 10 Iterationsstufen.

### Vorbehandeln der Substrate

Die eingesetzten Substrate aus Silizium, dem Hartmetall WC-Co, den Edelstählen Ti6A14V und einem chromhaltigen Edelstahl mit Beimengungen von Mn (Werkstoffnummer: 1.4034, X46Cr13) wurden je nach Art und Qualität der Oberfläche des Materials einer aufwändigen Prozedur des Schleifens und Polierens unterzogen.

Die Rauheit (Ra) der Substrate nach dem Schleifen und Polieren betrug:

| Substrat | Ra/nm | R_{Z(ISO)}/nm | Rt/nm |
|---|---|---|---|
| X46Cr13 | 11 | 50 | 40 |
| Ti6A14V | 12 | 60 | 65 |
| WC-Co | 2 | 12 | 15 |
| Silizium | 1 | 9 | 11 |

Die Substrate wurden vor der Beschichtung jeweils für 15 Minuten in einem Ultraschallbad in Aceton und anschließend in Isopropanol gereinigt. Danach wurden die Substrate mit destilliertem Wasser abgespült. Die Substrate wurden nur mit Latexhandschuhen angefasst, um das Übertragen von Schmutzpartikeln und Fett durch die Haut zu verhindern.

### Positionieren der Substrate in der Prozesskammer

Anschließend erfolgte die Positionierung der Substrate in der Prozesskammer einer Beschichtungsanlage (Magnetron-Sputter-Anlage). Die Evakuierung des Rezipienten wurde bis zu einem Restgasdruck von 2·10⁻⁴ Pa durchgeführt.

### Plasmaätzen der Substrate

Vor der eigentlichen Beschichtung wurden die Substrate in einem Plasmaätzverfahren gereinigt. Hierbei kamen zwei Verfahren zur Anwendung:
1.) *HF* (Hochfrequenz) *Plasmaätzprozess* bei 500 W. Als Arbeitsgas diente Argon, der Druck im Rezipienten betrug 0,6 Pa. Die Parameter wurden über eine Zeit von 20 Min. konstant gehalten.
2.) *DC-Glowing-Glimmentladung im DC-Feld (direct current)* bei einer Gleichspannung (DC-Spannung) von 500 bis 800 V. Als Arbeitsgas wurde ebenfalls Argon eingesetzt, der Druck im Rezipienten betrug auch hier 0,6 Pa. Die genannten Parameter wurden über eine Zeit von 15 bis 90 Minuten konstant gehalten.

Während der beschriebenen Verfahren wurden von der Substratoberfläche ca. 100 nm der Adsorbate bzw. des Substrates abgetragen und sowohl bei X46Cr13 als auch bei Ti6A14V ein Ra-Wert von 6 nm erzielt.

### Beschichten der Substrate

Die amorphen Kohlenstoffschichten wurden mittels Magnetronzerstäubung *(magnetron sputtering)* eines oder mehrerer Graphit-Targets in reiner Argon-Atmospähre bei 0,2 bis 2,0 Pa auf die so vorbehandelten und plasmageätzten Substrate bei Temperaturen bis 150°C abgeschieden. Es wurden sowohl Rundkathoden mit einem Durchmesser von 75 und 150 mm, als auch Rechteckkathoden mit Abmessungen von 135 x 465 mm oder 134 x 496 mm eingesetzt. Der Abstand zwischen Target und Substrat betrug je nach Beschichtungsanlage zwischen 50 und 150 mm. Für Kathoden mit einem Durchmesser von 75 mm wurden Sputterleistungen von 50 bis 500 W, für einen Durchmesser von 150 mm von 100 bis 1000 W und für die Rechteckkathoden von 1500 bis 6000 W gefahren. Die Leistungsdichte (Sputterleistung pro Flächeneinheit) lag demnach insgesamt zwischen 0,57 und 13,58 W/cm², wobei eine Leistungsdichte von höchstens 7,91 W/cm² erforderlich war, um eine thrombozytenanreichernde Schicht zu erhalten, und eine Leistungsdichte von mindestens 7,92 W/cm² erforderlich war, um eine thrombozytenabweisende Schicht zu erhalten.

Die Schichten wurden mit HF-Substratvorspannungen von 0 V bis -200 V abgeschieden, wobei diese im Bereich bis -200 V die Leistungsdichte nicht signifikant beeinflusst. Der Wert des Druckes hat nur bei niedrigen Leistungsdichten den Effekt, die Oberflächenenergie über den Schwellenwert von 34,4 mN/m zu heben. Damit die Oberflächenenergie unterhalb des Schwellenwerts von 34,4 mN/m bleibt, muss bei niedrigen Leistungsdichten ein geringer Druck gewählt werden.

## Patentansprüche

1. Verfahren zur Herstellung einer thrombozytenabweisenden amorphen Kohlenstoffschicht, die einen Gehalt an Wasserstoff von höchstens 5 Atom% aufweist, auf einem Substrat, mit den **Schritten:**
a) Schleifen, Polieren und Reinigen des Substrats,
b) Positionieren des Substrats in einer Prozesskammer einer Magnetronsputteranlage und Evakuierung der Prozesskammer auf einen Restgasdruck von 1·10⁻⁴ Pa bis 4·10⁻⁴ Pa,
c) Plasmaätzen des Substrats,
d) Abscheiden der amorphen Kohlenstoffschicht von einem Graphit-Target auf das so behandelte Substrat bei einer Sputterleistung pro Flächeneinheit von 7,91 bis 15,58 W/cm², bei einer Temperatur von 50 °C bis 200 °C und bei einem Argon-Partialdruck von 0,2 Pa bis 2,0 Pa.

2. Verfahren nach Anspruch 1, wobei ein Substrat mit einer Oberfläche aus einem Metall, einer Legierung, aus Glas, einer Keramik oder aus einem Polymer eingesetzt wird.

## Claims

1. Method for producing a thrombocyte-repelling amorphous carbon layer, which has a hydrogen content of at most 5 at%, on a substrate, comprising the steps:
a) grinding, polishing and cleaning the substrate,
b) positioning the substrate in a process chamber of a magnetron sputtering unit and evacuating the process chamber to a residual gas pressure of 1·10⁻⁴ Pa to 4·10⁻⁴ Pa,
c) carrying out plasma etching of the substrate,
d) depositing the amorphous carbon layer from a graphite target onto the treated substrate at a sputtering power per unit area of 7.91 to 15.58 W/cm², at a temperature of 50°C to 200°C and at an argon partial pressure of 0.2 Pa to 2.0 Pa.

2. Method according to claim 1, wherein use is made of a substrate having a surface made of a metal, an alloy, glass, a ceramic or a polymer.

## Revendications

1. Procédé d'obtention d'une couche de carbone amorphe repoussant les trombocytes présentant une teneur en hydrogène d'au maximum 5 % atomique, sur un substrat, comportant les étapes consistant à :
a) poncer, polir et nettoyer le substrat,
b) positionner le substrat dans une chambre de procédé d'une installation de pulvérisation à magnétron et mise sous vide de la chambre de procédé à une pression de gaz résiduelle de 1.10⁻⁴ Pa à 4.10⁻⁴ Pa,
c) décapage du substrat au plasma,
d) dépôt de la couche de carbone amorphe à partir d'une cible de graphite sur le substrat ainsi traité avec une puissance de pulvérisation par unité de surface de 7,91 à 15,58 W/cm², à une température de 50°C à 200°C et sous une pression partielle d'argon de 0,2 Pa à 2,0 Pa.

2. Procédé conforme à la revendication 1, selon lequel on met en oeuvre un substrat ayant une surface en un métal, un alliage, un verre, une céramique ou un polymère.
